# EUROPÄISCHE PATENTSCHRIFT

(11) **EP 0 914 460 B1**
(45) Veröffentlichungstag und Bekanntmachung des Hinweises auf die Patenterteilung: **02.04.2003**
(21) Anmeldenummer: 97925841.5
(22) Anmeldetag: 07.05.1997
(51) Int. Cl.: C12N 15/87, C12N 15/86, A61K 48/00

(54) **VEHIKEL ZUM TRANSPORT VON MOLEKULARER SUBSTANZ**
VEHICLE FOR THE TRANSPORT OF MOLECULAR SUBSTANCES
VEHICULE POUR LE TRANSPORT D'UNE SUBSTANCE MOLECULAIRE

(30) Priorität: 10.05.1996 DE 19618797
(43) Veröffentlichungstag der Anmeldung: 12.05.1999
(73) Patentinhaber: november Aktiengesellschaft Gesellschaft für Molekulare Medizin, 91056 Erlangen (DE)
(72) Erfinder: Bertling, Wolf, 91056 Erlangen (DE)
(74) Vertreter: Gassner, Wolfgang, Dr.
(86) Internationale Anmeldenummer: DE9700919
(87) Internationale Veröffentlichungsnummer: WO97043431

(56) Entgegenhaltungen:
- EP-A- 0 259 149
- DE-A- 4 335 025
- GB-A- 2 257 431
- US-A- 4 950 599
- MOLECULAR IMMUNOLOGY, Bd. 28, Nr. 3, 1991, Seiten 269-278, XP002040017 MARK J. REDMOND ET AL.: "Rotavirus particles function as immunological carriers for the delivery of peptides from infectious agents and endogenous proteins"
- VIROLOGY, Bd. 194, Nr. 1, Mai 1993, ORLANDO US, Seiten 393-398, XP002040018 SUE E. DELOS ET AL.: "Expression of the Polyomavirus VP2 and VP3 proteins in insect cells: Coexpression with the major capsid protein VP1 alters VP2/VP3 subcellular localization"

## Beschreibung

Die Erfindung betrifft ein Verfahren zur Herstellung eines Vehikels und die Verwendung des gemäß dem erfindungemäßen Verfahren Hergstellten Partikels.

Eukaryontische Zellen nehmen unter bestimmten Bedingungen DNA, Proteine und andere Moleküle auf. Die Aufnahmerate ist allerdings meist gering. Außerdem ist der Transport der molekularen Substanz in bezug auf die Art der Zellen sowie die Zellkompartimente oder den Ort im Intrazellulärbereich nicht vorherbestimmbar.

Um insbesondere die Aufnahme von DNA in eukaryontische Zellen zu verbessern, ist es bekannt, virale Vektoren als Vehikel zum Transport in die Zelle zu verwenden. - Die Verwendung viraler Vektoren ist nachteilig, weil es dabei zur Kotransfektion viraler Genome kommen kann.

Aus der US 4,950,599 ist des weiteren bekannt, molekulare Substanz wie DNA unter Verwendung leerer Viruskapside, insbesondere Polyomakapside, in eukaryontische Zellen zu schleusen. - Auch bei diesem Verfahren kann eine Kotransfektion viraler Genome nicht ausgeschlossen werden. Außerdem können Moleküle, deren Größe das Innenvolumen des Polyomakapsids übertreffen, darin nicht verpackt werden. Schließlich ist eine synthetische Herstellung von Polyomakapsiden, die als Möglichkeit der Vermeidung einer Kotransfektion in Betracht kommt, äußerst schwierig und kostenaufwendig.

Aufgabe der Erfindung ist es, die Nachteile des Stands der Technik zu beseitigen, insbesondere ein Vehikel zum Transport molekularer Substanz in eukaryontische Zellen anzugeben, das universell verwendbar sowie einfach und kostengünstig herstellbar ist.

Diese Aufgabe wird durch die Merkmale der Ansprüche 1 und 16 gelöst. Vorteilhafte Ausgestaltungen der Erfindung ergeben sich aus den Merkmalen der Ansprüche 2 - 15.

Nach Maßgabe der Erfindung ist ein Verfahren zur Herstellung eines Vehikel vorgesehen, das mindestens ein von einem Virus abgeleitetes oder stammendes Kapsomer aufweist, das an seiner einen Seite eine mit der molekularen Substanz in Wechselwirkungen tretende Struktur aufweist, so daß die molekulare Substanz an das Kapsomer bind- bzw. anlagerbar ist.

Das Vehikel hat den Vorteil, daß es relativ einfach synthetisch herstellbar ist. Somit kann eine Kotransfektion viraler Genome vermieden werden. Außerdem kann wegen des Vorsehenes der mit der molekularen Substanz in Wechselwirkung tretenden Struktur molekulare Substanz jeglicher Größe gebunden und in Zellen geschleust werden. Dazu muß die typische Kapsidform nicht mehr gewahrt werden. Unter Verwendung der erfindungsgemäßen Vehikel bilden sich neben Kapsomeren auch andersartige schützende Formen aus. Ein besonderer Vorteil der Erfindung ist darin zu sehen, daß es mit dem Vehikel je nach Ausbildung des mindestens einen Kapsomers möglich ist, die molekulare Substanz spezifisch in bestimmte Zellen und/oder an einen vorgegebenen Ort im Intrazellulärbereich zu transportieren.

Das Kapsomer ist vorzugsweise so ausgebildet, daß es zum Aufbau eines Kapsids oder eines kapsidartigen Gebildes geeignet ist. Von besonderem Vorteil ist es, wenn das Kapsomer spontan Kapside bildet.

Nach einem weiteren Ausgestaltungsmerkmal der Erfindung ist das Kapsomer vom Polyomavirus abgeleitet, wobei es aus dem VP1-Pentamer des Polyomavirus gebildet sein kann.

Alternativ dazu kann das Kapsomer aus "non-enveloped" Viren, wie DNA-haltigen Papovaviridae, insbesondere Polyoma- und den Papillomaviren, Iridoviridae, Adenoviridae, Parvoviridae oder RNA-haltigen Picornaviridae, insbesondere Polioviren, Caliciviridae, Reoviridae und Birnaviridae, gewonnen oder davon abgeleitet sein. Je nach Art der zu transportierenden molekularen Substanz kann es auch von Vorteil sein, das Kapsomer aus der äußeren und/oder inneren Hülle von "enveloped" Viren wie DNA-haltigen Poxviridae, Herpesviridae, Hepadnaviridae oder RNA-haltigen Retroviridae, Paramyxoviridae, Sendaiviren, Orthomyxoviridae, Bunyaviridae, Arenaviridae, Toroviridae, Togaviridae, Flaviviridae, Rhabdoviridae und Filoviridae zu gewinnen oder davon abzuleiten.

Bei den Wechselwirkungen handelt es sich zweckmäßigerweise um lipophile Wechselwirkungen und/oder Wechselwirkungen, die auf kovalenten, ionischen oder Wasserstoffbrücken-Bindungen beruhen. Damit ist sichergestellt, daß die molekulare Substanz beim Transport in die Zelle sicher am Vehikel gebunden bzw. angehaftet bleibt, sich jedoch nach vollzogenem Transport in die Zelle vom Vehikel löst bzw. durch zelluläre Systeme abgelöst werden kann.

Die Struktur kann bifunktionelle, vorzugsweise heterolog bifunktionelle Gruppen umfassen, wobei die bifunktionellen Gruppen vorzugsweise aus der Stoffgruppe der Maleinimid-Derivate, Alkylhalide, Arylhalide, Isocyanate, Glutardialdehyde, acrylierenden Reagentien und Imidoester ausgewählt sind. Dadurch wird insbesondere die Abgabe der molekularen Substanz im Lysosom, im zytoplasmatischen Raum oder im Kern erreicht.

Als besonders zweckmäßig hat es sich erwiesen, daß die bifunktionellen Gruppen mit Cystein-Resten am Kapsomer reagieren. Als vorteilhaft wird des weiteren angesehen, daß die in Wechselwirkung tretende Struktur affinitätserhöhende Gruppen, wie 4-Iodoacetamido-salicylsäure und/oder p-Arsonsäurephenyldiazonium-fluoroborat und/oder Derivate davon, umfaßt. - Die Struktur kann auch durch Epitope des VP1-Pentamers gebildet sein.

Nach einer weiteren Ausgestaltung des erfindungsgemäßen Verfahrens kann auch ein vehikel bareitgestellt werden bei dem mit mindestens einem weiteren Kapsomer ein kapsidartiges Gebilde zum Transport der molekularen Substanz in eine vorgegebene Art von Zellen oder an einen vorgegebenen Ort im Intrazellulärbereich vorhanden ist. Die Wahl der Art der Kapsomere und deren Kombination zur Herstellung des kapsidartigen Gebildes hängt von der Art der Zelle bzw. vom vorgegebenen Ort im Intrazellulärbereich ab, in die bzw. an den die molekulare Sustanz transportiert werden soll.

Zweckmäßigerweise ist die eine Seite des Kapsomers Bestandteil der Innenseite des kapsidartigen Gebildes, wobei das kapisidartige Gebilde vorzugsweise vom Polyomavirus abgeleitet ist. Schließlich kann das kapsidartige Gebilde mindestens ein VP-2 und/oder VP-3 Protein umfassen.

Zur Herstellung des Vehikels ist ein die folgenden Schritte umfassendes Verfahren vorgesehen:
i) Synthetisieren, Aufreinigen oder Isolieren des Kapsomers und
ii) Komplexieren der molekularen Substanz unter Verwendung des Kapsomers.

Eine Weiterbildung des Verfahrens besteht darin, nach dem Schritt lit.i geeignete Reste des Kapsomers, insbesondere dessen Cystein-Reste, mit bifunktionellen Gruppen zu modifizieren. Die Modifizierung kann zweckmäßigerweise unter Verwendung einer oder mehrerer der folgenden Stoffe durchgeführt werden: Maleinimid-Derivate, Alkylhalide, Arylhalide, Isocyanate, Glutardialdehyde, acrylierende Reagenzien und Imidoester.

Das Vehikel kann vorzugsweise als Arzneistoffträger zur Applikation von Molekülen, wie DNA, RNA, Oligonukleotiden, PNA, Proteinen, Peptiden sowie von niedermolekularen lipophilen und lipophoben Reagenzien, von kolloidalem Gold, Gold-markierten Proteinen und Peptiden, in eukaryontische Zellen verwendet werden.

Eine Zusammenstellung des Vehikels mit zur Applikation geeigenten oder notwendigen Mitteln, bsp. Reagentien, Lösungsmitteln u.ä., ist möglich. Gleichfalls ist eine Zusammenstellung von Mitteln zur Durchführung des erfindungsgemäßen Verfahrens vorgesehen. Diese Zusammenstellung kann auch Geräte u. dgl. umfassen.

Die Erfindung wird anhand der folgenden Beispiele und Darstellungen näher beschrieben. Es zeigen
- Fig.1: den gelelektrophoretischen Nachweis der VP3, VP2 und VP1-Fusionsproteine,
- Fig.2: links eine elektronenmikroskopische Ansicht von aus dem VP1-Protein gebildeten Pentameren und rechts eine computerunterstützte Darstellung der 5-fachen Symmetrie der Pentamere,
- Fig.3: hergestellte Pentamere und daraus gebildete Kapside und
- Fig.4: eine elektronenmikroskopische Ansicht beladener VP1-Kapside.

Die nachfolgenden Beispiele beschreiben eine mögliche Ausführung der Erfindung.

### 1) Expression des VP1-Proteins von Polyomavirus in E.coli:

Es wird ein Gen des VP1 Hüllproteins des murinen Polyomavirus hergenommen, das sowohl Sequenzmerkmale des Stammes A2 als auch des Stammes A3 aufweist. Die kodierende Sequenz beginnend mit dem ATG bzw. der darauf folgenden Aminosäure wird unmittelbar hinter einer Faktor Xa Schnittstelle in ein Derivat des kommerziell erhältlichen Vektors pQE 10 der Firma Quiagen kloniert. Dieser Vektor versieht das Fusionsprotein Xa Schnittstelle-VP1 am Aminoterminus mit einer Histidinabfolge. Das so gewonnene Fusionskonstrukt ist innerhalb eines Markergens (lacZ-Komplementation) kloniert und ist über den lacZ Promotor induzierbar. Das Endkonstrukt wird in für die Expression von pQE-Vektoren geeignete E.coli Zellen transformiert. Wenn die Zellen nach vorheriger Anzüchtung in der logarithmischen Phase sind, werden sie durch Zugabe eines geeigneten Induktors, bsp. IPTG, induziert. Sie exprimieren daraufhin große Mengen eines das VP1-Protein enthaltenden Fusionsproteins. Das Fusionsprotein wird nach 6-stündiger Induktion geerntet. Es liegt in löslicher Form vor und kann ohne größere Änderungen des Aufreinigungsprotokolls der Firma Quiagen über Nickelchelatsäulen rein dargestellt werden. Durch Inkubation mit Faktor Xa kann der reine VP1-Proteinanteil des Fusionsproteins von der Nickelchelatsäule wieder abgetrennt werden. Das erhaltene VP1-Protein liegt in sehr reiner Form vor und bildet von sich aus Pentamere. Analog können die Proteine VP2 und VP3 dargestellt werden.

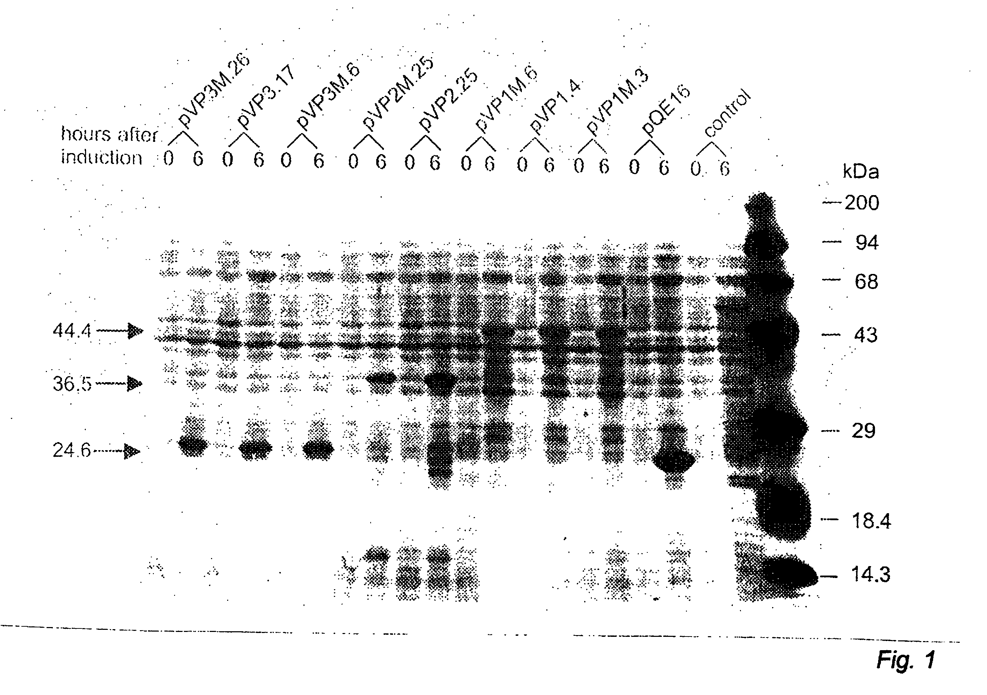

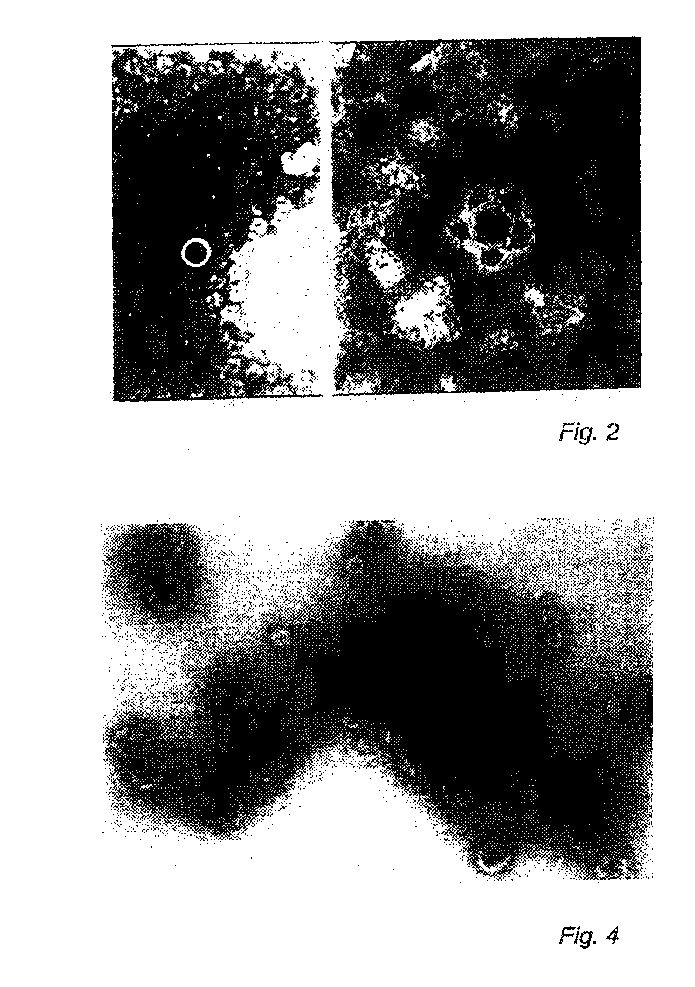

Die Fig. 1 zeigt den gelelektrophoretischen Nachweis der VP3, VP2 und VP1-Fusionsproteine. Aus Fig. 2 ist links eine elektronenmikroskopische Ansicht von aus dem VP1-Protein gebildeten Pentameren und rechts eine computergestützte Darstellung der 5-fach Symmetrie der Pentamere ersichtlich.

### 2) Modifikation der Cystein-Reste an der einen Seite der Pentamere vor deren Assemblierung:

Die gemäß Ziffer 1 gewonnenen VP1-Pentamere besitzen mehrere Strukturen, die durch Reaktion mit geeigneten Reagenzien in bifunktionelle Gruppen umwandelbar sind. Die Strukturen befinden sich auf der Seite der Pentamere, die nach Assemblierung zum Kapsid dessen Innenseite entspricht. Als Reagenz wird ein in einer Aceton-Methanol-Wasser-Mischung dispergierter 3-Maleinimidobenzoyl-N-hydroxy-succinimidester verwendet, der auf der einen Seite des Reaktionszentrums als reaktive Gruppen SH-Gruppen und auf der anderen Seite eine Reaktivestergruppe, nämlich einen aminogruppenreaktiven Succinimidester, trägt. Die Dispersion wird mit den gelösten VP1-Proteinen gemischt, so daß eine quantitative Umsetzung erfolgt.

Aus Tabelle 1 sind die Loop-Strukturen von Polyomakapsomeren ersichtlich, die auf der einen Seite der Kapsomere zu finden sind, die nach der Assemblierung zur Innenseite des Kapsids bzw. der kapsidartigen Struktur weisen:

**Tabelle 1**

| | |
|---|---|
| Loop 1 | Asp 38, Leu 39, Val 40, Thr 41, Gly 42, Pro 43, Asp 44, Ser 45 |
| Loop 2 | Asn 109, Glu 110, Asp 111, Leu 112, Thr 113, Lys 114, Asp 115, Thr 116, Leu 117 |
| Tail | N-Terminus von Aminosäurerest 1 bis Rest 29 (zumindest aber ab der in der Strukturanalyse gut lokaliesierten Aminosäure 18 vom N-Terminus bis zu Rest 29): Lys 18, Ala 19, Cys 20, Pro 21, Arg 22, Pro 23, Ala 24, Pro 25, Val 26, Pro 27, Lys 28, Leu 29 |
| Loop 3 | Tyr 354, Asp 355, Gly 356, Thr 357, Gln 358, Pro 359, Val 360 |

### 3) Die Assemblierung von VP1-Pentameren zu VP1-Kapsiden:

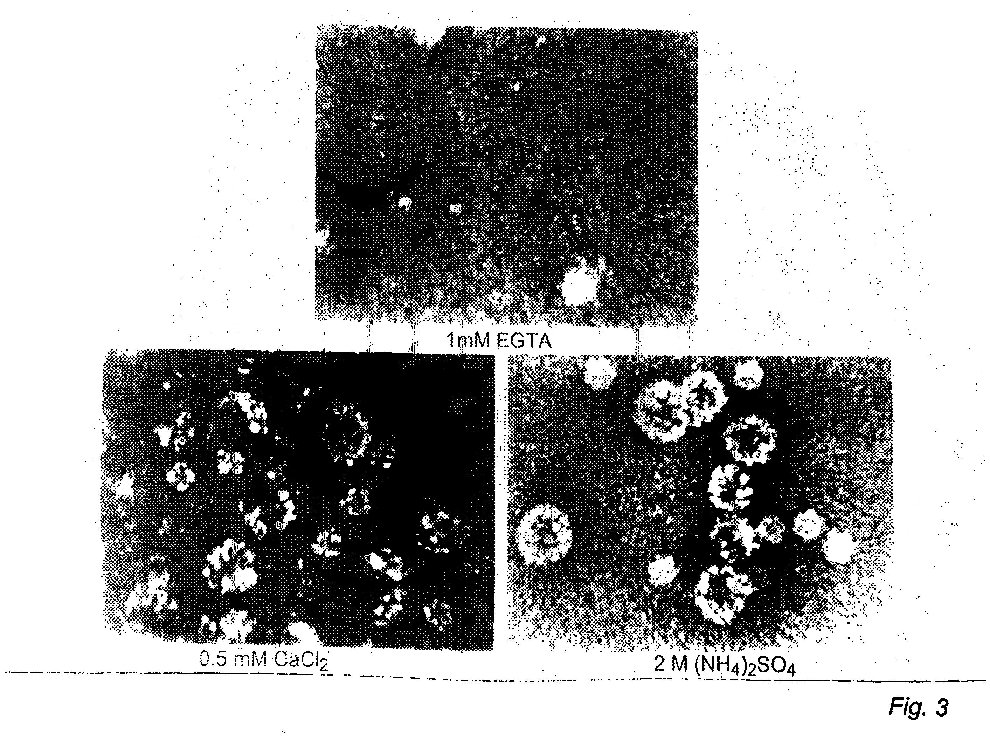

Die VP1-Pentamere liegen in einer Pufferlösung vor, die EGTA zur Stabilisierung des pentameren nicht assemblierten Zustands enthält. Ferner sind der Pufferlösung Magnesium-Ionen, Natrium-Ionen und Tris/HCl, pH 7,6, zur Stabilisierung des pH zugesetzt. Die Proteinlösung wird in eine Dialysekammer überführt und gegen eine 2M Ammoniumsulfatlösung dialysiert. Nach mehrfachem Wechsel des Dialysepuffers bilden die VP1-Pentamere Kapside. Diese unterscheiden sich von leeren Kapsiden des Polyomavirus weder bei Betrachtung im Elektronenmikroskop noch im Durchmesser, noch in ihrer Stabilität, obwohl ihnen die inneren Hüllproteine VP2 und VP3 fehlen. Fig. 3 zeigt die hergestellten Pentamere und daraus gebildete Kapside.

### 4) Die Verpackung von DNA Oligonukleotiden in Polyoma-VP1 Kapside:

Konventionelle, d.h. in ihrer chemischen Struktur nicht veränderte Oligonukleotide, lassen sich nach folgendem Protokoll mit hoher Ausbeute in Polyoma-VP1 Kapside verpacken: Kapsidstrukturen, wie sie im Beispiel 3 gewonnen worden sind, werden auf pH 5,5 umgepuffert. Anschließend werden sie in einer osmotischen Schockprozedur mit einer equi- oder höher molaren Menge, typischerweise mit einem zweifachen molaren Überschuß an Oligonukleotiden umgesetzt. Für die in diesem Beispiel verwendeten Oligonukleotide (20-mere) ergibt sich damit ein Gewichtsverhältnis von ca. 1:6 gegenüber dem VP1-Protein. Die Form der so erhaltenen mit Oligonukleotiden beladenen VP1-Kapside läßt sich im Elektronenmikroskop nicht von der unbeladener VP1-Kapside unterscheiden. Fig. 4 zeigt eine elektronenmikroskopische Ansicht beladener VP1-Kapside.

## Patentansprüche

1. Verfahren zur Herstellung eines Vehikels mit molekularer Substanz, wie DNA, RNA, Protein, PNA, Arzneistoffe lipophilen und lipophoben Charakters, zum Transport in eukaryontische Zellen, umfassend mindestens ein von einem Virus abgeleitetes oder stammendes Kapsomer, das so ausgebildet ist, daß es zum Aufbau eines Kapsids oder eines kapsidartigen Gebildes geeignet ist und an seiner einen Seite eine mit der molekularen Substanz in Wechselwirkungen tretende Struktur aufweist, so daß die molekulare Substanz an das Kapsomer bind- bzw. anlagerbar ist, und wobei die eine Seite des Kapsomers nach Assemblierung Bestandteil der Innenseite des Kapsids bzw. kapsidartigen Gebildes ist, umfassend die folgenden Schritte:
i) Synthetisieren, Aufreinigen oder Isolieren des Kapsomers und
ii) Komplexieren der molekularen Substanz unter Verwendung des Kapsomers.

2. Verfahren nach Anspruch 1, wobei das Kapsomer vom Polyomavirus abgeleitet ist.

3. Verfahren nach Anspruch 2, wobei das Kapsomer aus dem VP1-Pentamer des Polyomavirus gebildet oder davon abgeleitet ist.

4. Verfahren nach Anspruch 1, wobei das Kapsomer aus "non-enveloped" Viren wie DNA-haltigen Papovaviridae, insbesondere Polyoma- und Papillomaviren, Iridoviridae, Adenoviridae, Parvoviridae oder RNA-haltigen Picornaviridae, insbesondere Polioviren, Caliciviridae, Reoviridae und Birnaviridae gewonnen oder davon abgeleitet ist.

5. Verfahren nach Anspruch 1, wobei das Kapsomer aus der äußeren und/oder inneren Hülle von "enveloped" Viren wie DNA-haltigen Poxviridae, Herpesviridae, Hepadnaviridae oder RNA-haltigen Retroviridae, Paramyxoviridae, Sendaiviren, Orthomyxoviridae, Bunyaviridae, Arenaviridae, Toroviridae, Togaviridae, Flaviviridae, Rhabdoviridae und Filoviridae gewonnen bzw. davon abgeleitet ist.

6. Verfahren nach einem der vorhergehenden Ansprüche, wobei die Wechselwirkungen lipophile Wechselwirkungen sind und/oder auf kovalenten, ionischen oder Wasserstoffbrücken-Bindungen beruhen.

7. Verfahren nach einem der vorhergehenden Ansprüche, wobei die in Wechselwirkung tretende Struktur bifunktionelle, vorzugsweise heterolog bifunktionelle Gruppen, umfaßt.

8. Verfahren nach Anspruch 7, wobei die bifunktionellen Gruppen aus der Stoffgruppe der Maleinimid-Derivate, Alkylhalide, Arylhalide, Isocyanate, Glutardialdehyde, acrylierenden Reagenzien und Imidoester ausgewählt sind.

9. Verfahren nach Anspruch 7 oder 8, wobei die bifunktionellen Gruppen mit Cystein-Resten am Kapsomer reagieren.

10. Verfahren nach einem der vorhergehenden Ansprüche, wobei die in Wechselwirkung tretende Struktur affinitätserhöhende Gruppen, wie 4-Iodoacetamide-salicylsäure und/oder p-Arsonsäure-phenyldiazoniumfluoroborat und/oder Derivate davon, umfaßt.

11. Verfahren nach einem der Ansprüche 3 bis 10, wobei die in Wechselwirkung tretende Struktur durch Epitope des VP1-Pentamers gebildet ist.

12. Verfahren nach einem der vorhergehenden Ansprüche, wobei mit mindestens einem weiteren Kapsomer ein kapsidartiges Gebilde zum Transport der molekularen Substanz in eine vorgegebene Art von Zellen oder an einen vorgegebenen Ort im Intrazellulärbereich herstellbar ist.

13. Verfahren nach einem der vorhergehenden Ansprüche, wobei das kapsidartige Gebilde mindestens ein VP-2 und/oder VP-3 Protein umfaßt.

14. Verfahren nach einem der vorhergehenden Ansprüche, wobei nach dem Schritt lit.i die geeigneten Reste des Kapsomers, insbesondere dessen Cystein-Reste, mit bifunktionellen Gruppen modifiziert werden.

15. Verfahren nach einem der vorhergehenden Anspüche, wobei die Modifikation unter Verwendung einer oder mehrerer der folgenden Stoffe durchgeführt wird: Maleinimid-Derivate, Alkylhalide, Arylhalide, Isocyanate, Glutardialdehyde, acrylierenden Reagentien und Imidoester.

16. Verwendung des nach dem Verfahren nach einem der vorhergehenden Ansprüche hergestellten Vehikels als Arzneistoffträger zur Applikation von Molekülen wie DNA, RNA, Oligonukleotiden, PNA, Proteinen, Peptiden sowie niedermolekularen lipophilen und lipophoben Reagenzien, kolloidalem Gold und Gold-markierten Proteinen und Peptiden in eukaryontische Zellen.

## Claims

1. A process for the manufacture of a vehicle with molecular substance, such as DNA, RNA, protein, PNA, pharmaceuticals of lipophilic and lipophobic character, for transport into eukaryotic cells, comprising at least one capsomere derived or originating from a virus, which is constructed so that it is suitable for the construction of a capsid or a capsid-like structure and on its one side has a structure coming to interact with the molecular substance so that the molecular substance can be bound or added to the capsomere, and whereby after assembly the one side of the capsomere is a constituent of the inner side of the capsid or capsid-like structure, comprising the following steps:
i) synthesis, purification or isolation of the capsomere and
ii) complexing of the molecular substance using the capsomere.

2. A process according to Claim 1, whereby the capsomere is derived from the Polyoma virus.

3. A process according to Claim 2, whereby the capsomere is formed from the VP1 pentamer of the Polyoma virus or is derived therefrom.

4. A process according to Claim 1, whereby the capsomere is obtained from "non-enveloped" viruses such as DNA-containing Papovaviridae, in particular Polyoma and Papilloma viruses, Iridoviridae, Adenoviridae, Parvoviridae or RNA-containing Picornaviridae, in particular polio viruses, Calicivirdae, Reoviridae and Birnaviridae or is derived therefrom.

5. A process according to Claim 1, whereby the capsomere is obtained from the outer and/or inner coat of "enveloped" viruses such as DNA-containing Poxviridae, Herpesviridae, Hepadnaviridae or RNA-containing Retroviridae, Paramyxoviridae, Sendai viruses, Orthomyxoviridae, Bunyaviridae, Arenaviridae, Toroviridae, Togaviridae, Flaviviridae, Rhabdoviridae and Filoviridae or is derived therefrom.

6. A process according to one of the preceding Claims, whereby the interactions are lipophile interactions and/or are based on covalent, ionic or hydrogenbridge bonds.

7. A process according to one of the preceding Claims, whereby the structure involved in the interaction comprises bi-functional, preferably heterologously bi-functional groups.

8. A process according to Claim 7, whereby the bi-functional groups are selected from the substance group of the maleinimide derivates, alkyl halides, aryl halides, isocyanates, glutaraldehydes, acrylating reagents and imidoesters.

9. A process according to Claim 7 or 8, whereby the bi-functional groups react with cysteine residues on the capsomere.

10. A process according to one of the preceding Claims, whereby the structure involved in the interaction comprises affinity-increasing groups, such as 4-iodoacetamidosalicylic acid and/or p-arsonic acid phenyldiazonium fluoroborate and/or derivatives thereof.

11. A process according to one of Claims 3 to 10, whereby the structure involved in the interaction is formed by epitopes of the VP1 pentamer.

12. A process according to one of the preceding Claims, whereby, using at least one further capsomere, it is possible to prepare a capsid-like structure for the transport of the molecular substance into a predetermined type of cell or at a predetermined site in the intracellular region.

13. A process according to one of the preceding Claims, whereby the capsid-like structure comprises at least one VP-2 and/or VP-3 protein.

14. A process according to one of the preceding Claims, whereby after step i the suitable residues of the capsomere, in particular its cysteine residues, are modified with bi-functional groups.

15. A process according to one of the preceding Claims, whereby the modification is carried out using one or more of the following substances: maleinimide derivatives, alkyl halides, aryl halides, isocyanates, glutar aldehydes, acrylating reagents and imidoesters.

16. Use of the vehicle produced in accordance with the process according to one of the preceding Claims as a pharmaceutical carrier for the administration of molecules, such as DNA, RNA, oligonucleotides, PNA, proteins, peptides, and also low-molecular-weight lipophile and lipophobic reagents, colloidal gold and gold-labelled proteins and peptides into eukaryotic cells.

## Revendications

1. Procédé de préparation d'un vecteur comprenant une substance moléculaire, comme l'ADN, l'ARN, une protéine, le PNA, des médicaments à caractère lipophile et lipophobe, destiné au transport dans des cellules eucaryotes, comprenant au moins un capsomère dérivé ou provenant d'un virus, qui est conçu de façon telle qu'il est approprié pour construire une capside ou un produit de type capside et présente sur l'un de ses côtés une structure interagissant avec la substance moléculaire, de sorte que la substance moléculaire peut être liée au capsomère ou, selon les cas, fixée à celui-ci, et où un côté du capsomère forme, après assemblage, un composant de la face interne de la capside ou du produit de type capside, comprenant les étapes suivantes :
i) la synthèse, la purification ou l'isolement du capsomère, et
ii) la complexation de la substance moléculaire en utilisant le capsomère.

2. Procédé selon la revendication 1, dans lequel le capsomère est dérivé du virus du polyome.

3. Procédé selon la revendication 2, dans lequel le capsomère est formé à partir du pentamère VP1 du virus du polyome ou est dérivé de celui-ci.

4. Procédé selon la revendication 1, dans lequel le capsomère est obtenu à partir de virus "non enveloppés", tels que les Papovaviridés contenant de l'ADN, en particulier les virus du polyome et du papillome, les Iridoviridés, les Adenoviridés, les Parvoviridés, ou les Picornaviridés contenant de l'ARN, en particulier les virus poliomyélitiques, les Caliciviridés, les Réoviridés et les Birnaviridés, ou est dérivé de ceux-ci.

5. Procédé selon la revendication 1, dans lequel le capsomère est obtenu à partir de l'enveloppe externe et/ou interne de virus "enveloppés", tels que les Poxviridés contenant de l'ADN, les Herpesviridés, les Hepadnaviridés ou les Rétroviridés contenant de l'ARN, les Paramyxoviridés, les virus de Sendai, les Orthomyxoviridés, les Bunyaviridés, les Arenaviridés, les Toroviridés, les Togaviridés, les Flaviviridés, les Rhabdoviridés et les Filoviridés, ou est dérivé de ceux-ci.

6. Procédé selon l'une des revendications précédentes, dans lequel les interactions sont des interactions lipophiles et/ou sont basées sur des liaisons covalentes, ioniques ou des ponts d'hydrogène.

7. Procédé selon l'une des revendications précédentes, dans lequel la structure à interactions comprend des groupements difonctionnels, de préférence hétérologues difonctionnels.

8. Procédé selon la revendication 7, dans lequel les groupements difonctionnels sont sélectionnés dans le groupe de substances composé des dérivés de l'imide maléique, des halogénures d'alkyle, des halogénures d'aryle, des isocyanates, des dialdéhydes glutariques, des réactifs à action acrylisante et des imidoesters.

9. Procédé selon la revendication 7 ou 8, dans lequel les groupements difonctionnels réagissent avec des radicaux cystéine sur le capsomère.

10. Procédé selon l'une des revendications précédentes, dans lequel la structure à interactions comprend des groupements augmentant l'affinité, tels que l'acide 4-iodoacétamidosalicylique et/ou le phényldiazoniumfluoroborate de l'acide p-arsonique, et/ou leurs dérivés.

11. Procédé selon l'une des revendications 3 à 10, dans lequel la structure à interactions est formée par des épitopes du pentamère VP1.

12. Procédé selon l'une des revendications précédentes, dans lequel, en utilisant au moins un autre capsomère, on peut préparer un produit de type capside pour le transport de la substance moléculaire dans un type donné de cellules ou à un endroit donné d'un domaine intracellulaire.

13. Procédé selon l'une des revendications précédentes, dans lequel le produit de type capside comprend au moins une protéine VP-2 et/ou VP-3.

14. Procédé selon l'une des revendications précédentes, dans lequel, après l'étape i.), les radicaux appropriés du capsomère, en particulier ses radicaux cystéine, sont modifiés avec des groupements difonctionnels.

15. Procédé selon l'une des revendications précédentes, dans lequel la modification est réalisée en utilisant une ou plusieurs des substances suivantes : dérivés de l'imide maléique, halogénures d'alkyle, halogénures d'aryle, isocyanates, dialdéhydes glutariques, réactifs à action acrylisante et imidoesters.

16. Utilisation du vecteur préparé selon le procédé en accord avec l'une quelconque des revendications précédentes comme support de médicament destiné à appliquer dans des cellules eucaryotes des molécules telles que de l'ADN, de l'ARN, des oligonucléotides, du PNA, des protéines, des peptides, ainsi que des réactifs lipophiles et lipophobes de faible masse moléculaire, de l'or colloïdal et des protéines et peptides marqués à l'or.
